# FASCICULE DE BREVET EUROPEEN

(11) **EP 1 446 402 B1**
(45) Date de publication et mention de la délivrance du brevet: **02.11.2005**
(21) Numéro de dépôt: 02793211.0
(22) Date de dépôt: 07.11.2002
(51) Int. Cl.: C07D 417/06, C07D 277/28, A61K 31/427, A61K 31/426, A61K 31/4439, A61K 31/506, A61P 25/16, C07D 333/20, C07D 413/06

(54) **DERIVES DE 2-AMINO-4-HETEROARYLETHYL THIAZOLINE ET LEUR UTILISATION COMME INHIBITEURS DE NO-SYNTHASE INDUCTIBLE**
2-AMINO-4-HETEROARYLETHYL-THIAZOLINDERIVATE UND IHRE VERWENDUNG ALS HEMMSTOFFE DER INDUZIERBAREN NO-SYNTHASE
2-AMINO-4-HETEROARYLETHYL THIAZOLINE DERIVATIVES AND THEIR USE AN INHIBITORS OF INDUCIBLE NO-SYNTHASE

(30) Priorité: 09.11.2001 FR 0114509; 30.01.2002 US 352977 P
(43) Date de publication de la demande: 18.08.2004
(73) Titulaire: Aventis Pharma S.A., 92160 Antony (FR)
(72) Inventeur: BACQUE, Eric, F-91190 Gif sur Yvette (FR); BIGOT, Antony, F-91300 Massy (FR); CARRY, Jean-Christophe, F-94100 Saint Maur des Fosses (FR); MIGNANI, Serge, F-92290 Chatenay Malabry (FR)
(74) Mandataire: Rousseau, Pierrick Edouard
(86) Numéro de dépôt international: PCT/FR2002/003809
(87) Numéro de publication internationale: WO 2003/040142

(56) Documents cités:
- WO-A-01/94325
- WO-A-94/12165
- WO-A-95/11231
- WO-A-96/14842
- CHEMICAL ABSTRACTS, vol. 54, no. 11, 10 juin 1960 (1960-06-10) Columbus, Ohio, US; abstract no. 11021h, NOIKE Y: "Syntheses of quinolizine derivatives. VI. Syntheses of 3-aminoquinolizines. 1. Syntheses of dl-3-amino, dl-3-epiamino-, and dl-3-epidimethylaminoquinolizidines" XP002110770 & YAKUGAKU ZASSHI, vol. 79, 1959, pages 1514-1518,
- SABAT M ET AL: "Synthesis of unnatural amino acids via Suzuki cross-coupling of enantiopure vinyloxazolidine derivatives" ORGANIC LETTERS, vol. 2, no. 8, 20 avril 2000 (2000-04-20), pages 1089-1092, XP002205081

## Description

La présente invention concerne l'utilisation de dérivés de 2-amino-4-hétéroaryléthyl-thiazoline de formule : 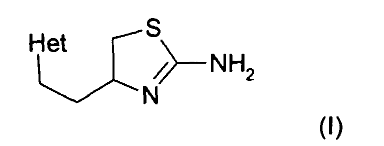 ou leurs sels pharmaceutiquement acceptables comme inhibiteurs de NO-synthase inductible.

L'invention a pour objet l'utilisation des dérivés de 2-amino-4-hétéroaryléthyl-thiazoline de formule (I) et leurs sels pharmaceutiquement acceptables pour la préparation de compositions pharmaceutiques destinées à prévenir et traiter les maladies dans lesquelles une production anormale de monoxyde d'azote (NO) par induction de NO-synthase inductible (NOS-2 ou iNOS) est impliquée, les compositions pharmaceutiques contenant les nouveaux dérivés de 2-amino-4-hétéroaryléthyl-thiazoline et leurs sels pharmaceutiquement acceptables et les dérivés nouveaux de 2-amino-4-hétéroaryléthyl-thiazoline et leurs sels pharmaceutiquement acceptables.

Le monoxyde d'azote (NO) est un radical diffusible impliqué dans de nombreux processus physiologiques et pathologiques. Il est synthétisé par oxydation de la L-arginine, une réaction catalysée par une famille d'enzymes appelée synthase du monoxyde d'azote ou NO-Synthase (NOS), référencée dans le système international de nomenclature des enzymes sous le numéro E.C. 1.14.13.39.

Trois isoformes de NOS, dont deux sont constitutives et une inductible, sont connues :
- une NOS neuronale (NOS-1 ou nNOS) a été isolée et clonée à l'origine à partir de tissu nerveux où c'est une enzyme constitutive. La NOS-1 produit du NO en réponse à divers stimuli physiologiques tels que l'activation de récepteurs membranaires selon un mécanisme dépendant du calcium et de la calmoduline.
- une NOS inductible (NOS-2 ou iNOS) peut être induite en réponse à des stimuli immunologiques tels que par exemple des cytokines ou des antigènes bactériens dans différentes cellules tels que par exemple les macrophages, les cellules endothéliales, les hépatocytes, les cellules gliales, ainsi qu'un grand nombre d'autres types de cellules. L'activité de cette isoforme n'est pas régulée par le calcium. C'est pourquoi une fois induite elle produit de grandes quantités de NO sur des durées prolongées.
- une NOS endothéliale (NOS-3 ou eNOS) est constitutive et calcium/calmoduline dépendante. Elle a été identifiée à l'origine dans les cellules de l'endothélium vasculaire où elle génère du NO en réponse à des stimuli physiologiques tels que l'activation de récepteurs membranaires.

Le NO produit par les isoformes constitutives neuronales et endothéliales (NOS-1 et NOS-3) est généralement impliqué dans des fonctions de signalisation intercellulaire. Par exemple, les cellules endothéliales qui tapissent la paroi interne des vaisseaux sanguins induisent la relaxation des cellules musculaires lisses sous-jacentes *via* la production de NO. Il contribue ainsi à la régulation de la pression artérielle.

Le NO produit en grande quantité par l'isoforme inductible NOS-2 est, entre autre, impliqué dans les phénomènes pathologiques associés aux processus inflammatoires aigus et chroniques dans une grande variété de tissu et d'organes.

Une production excessive de NO par induction de NOS-2 participe ainsi de pathologies dégénératives du système nerveux comme par exemple la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie. De même, en dehors du système nerveux central, l'induction de NOS-2 est impliquée dans de nombreuses pathologies à composantes inflammatoires comme par exemple le diabète, l'athérosclérose, la myocardite, l'arthrite, l'arthrose, l'asthme, le syndrome du colon irritable, la maladie de Crohn, la péritonite, le reflux gastro-oesophagien, l'uvéite, le syndrome de Guillain-Barré, la glomérulo-néphrite, le lupus érythémateux, le psoriasis. La NOS-2 a également été impliquée dans la croissance de certaines formes de tumeurs comme par exemple des épithéliomes, des adénocarcinomes ou des sarcomes, et dans les infections par des bactéries intracellulaires ou extracellulaires, Gram-plus ou Gram-moins.

Dans toutes les situations où une surproduction de NO est néfaste, il apparaît donc souhaitable de diminuer la production de NO par l'administration de substances capables d'inhiber la NOS-2. Cependant, compte tenu des rôles physiologiques importants joués par l'isoforme constitutive NOS-3 en particulier dans la régulation de la pression artérielle, il est primordial que l'inhibition de l'isoforme NOS-2 affecte le moins possible l'isoforme NOS-3. En effet, il est connu que l'administration d'inhibiteurs non-sélectifs des isoformes de NOS conduit à une vasoconstriction et à un accroissement de la pression artérielle (Moncada, S., Palmer, R.M.J. et Higgs, E.A., Biosynthesis of nitric oxide from L-arginine : a pathway for the regulation of cell function and communication, *Biochem. Pharmacol.,* 1989, 38: 1709-1715). Ces effets sur le système cardiovasculaire sont délétères dans la mesure où ils diminuent l'apport en nutriments aux tissus. Par conséquent, la présente invention concerne des composés présentant une activité inhibitrice vis-à-vis de la NOS-2 significativement plus puissante que son activité inhibitrice vis-à-vis de la NOS-3.

Des inhibiteurs de NOS dérivés de thiazoline sont notamment décrits dans les demandes de brevet WO94/12165, WO95/11231 et WO96/14842.

La présente invention concerne l'utilisation des dérivés de 2-amino-4-hétéroaryléthyl-thiazoline de formule (I) dans laquelle :

Het représente un radical 2-thiényle, 3-thiényle, 2-pyrimidyle, 5-pyrimidyle, 2-pyridyle, 3-pyridyle, 4-pyridyle, 2-thiazolyle, 4-thiazolyle ou 5-thiazolyle pour la préparation de médicaments utiles pour prévenir ou traiter les maladies dans lesquelles une production anormale de monoxyde d'azote (NO) par induction de NO-synthase inductible (NOS-2 ou iNOS) est impliquée.

Les composés de formule (I) présentent un ou plusieurs carbones asymétriques et peuvent donc se présenter sous forme de racémique, d'énantiomères et de diastéréoisomères; ceux-ci font également partie de l'invention ainsi que leurs mélanges.

Par ailleurs les composés de formule (I) peuvent se présenter sous la forme tautomère (Ia) :

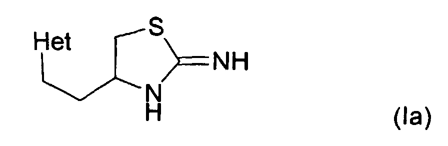

Ces tautomères font également partie de l'invention.

Parmi les composés de formule (I) utiles selon l'invention on peut citer les composés suivants:
4-(2-pyridin-2-yléthyl)-4,5-dihydro-1,3-thiazol-2-ylamine
4-(2-pyridin-3-yléthyl)-4,5-dihydro-1,3-thiazol-2-ylamine
4-(2-pyridin-4-yléthyl)-4,5-dihydro-1,3-thiazol-2-ylamine
4-(2-thién-3-yléthyl)-4,5-dihydro-1,3-thiazol-2-ylamine
leurs racémiques, énantiomères, diastéréoisomères, tautomères ainsi que leurs sels pharmaceutiquement acceptables,
et plus particulièrement les composés suivants :
(+)-(4R)-4-(2-pyridin-2-yléthyl)-4,5-dihydro-1,3-thiazol-2-ylamine
(+)-(4R)-4-(2-pyridin-3-yléthyl)-4,5-dihydro-1,3-thiazol-2-ylamine
(+)-(4R)-4-(2-pyridin-4-yléthyl)-4,5-dihydro-1,3-thiazol-2-ylamine
(4R)-4-(2-thién-3-yléthyl)-4,5-dihydro-1,3-thiazol-2-ylamine
leurs tautomères ainsi que leurs sels pharmaceutiquement acceptables.

Parmi les composés utiles selon l'invention et particulièrement préférés on peut citer le composé suivant :
4-(2-thién-3-yléthyl)-4,5-dihydro-1,3-thiazol-2-ylamine
son racémique, ses énantiomères, tautomères ainsi que leurs sels pharmaceutiquement acceptables,
et plus particulièrement le composé suivant :
(4R)-4-(2-thién-3-yléthyl)-4,5-dihydro-1,3-thiazol-2-ylamine
ses tautomères ainsi que ses sels pharmaceutiquement acceptables.

L'invention concerne également les compositions pharmaceutiques contenant en tant que principe actif un dérivé de formule (I) pour lequel Het représente un radical 2-thiényle, 3-thiényle, 2-pyrimidyle, 5-pyrimidyle, 2-pyridyle, 3-pyridyle, 4-pyridyle, 2-thiazolyle, 4-thiazolyle ou 5-thiazolyle ainsi que ses racémiques, énantiomères, diastéréoisomères et leurs mélanges, ses tautomères et ses sels pharmaceutiquement acceptables.

Les composés de formule (I) peuvent être préparés par cyclisation d'un dérivé de formule : 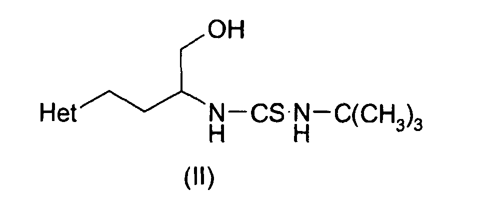 dans laquelle Het a la même signification que dans la formule (I).

Cette cyclisation s'effectue généralement au moyen d'un acide tel que l'acide chlorhydrique, en milieu aqueux, à une température voisine de 100° C. On utilise en général l'acide chlorhydrique 6N.

Les dérivés de formule (II) peuvent être obtenus selon le schéma réactionnel suivant : 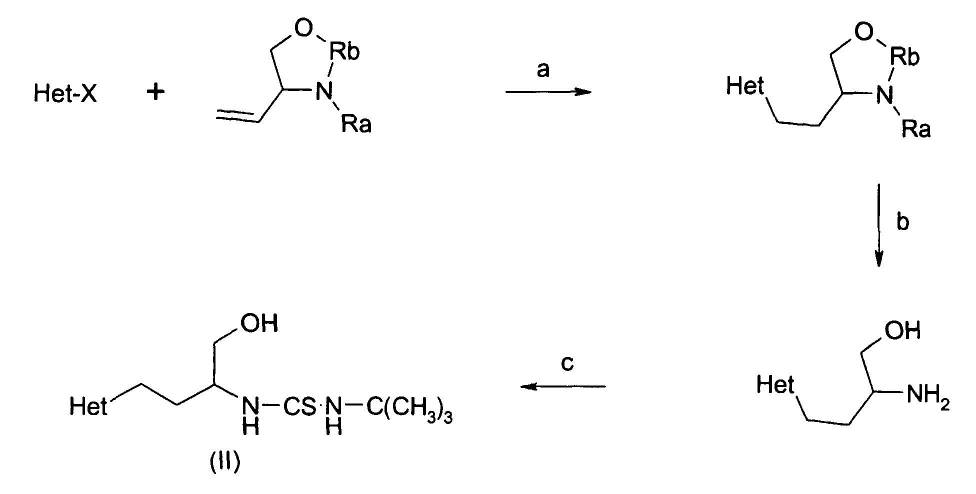 dans ces formules Het a les mêmes significations que dans la formule (I), Ra est un groupe protecteur de la fonction amine tel que ceux décrits par T.W. GREENE, Protective groups in Organic Synthesis, J. Wiley-Interscience Publication (1991) et Rb est un groupe protecteur de la fonction β-amino alcool tel que ceux décrits par T.W. GREENE, Protective groups in Organic Synthesis, J. Wiley-Interscience Publication (1991). De préférence le groupe protecteur de la fonction amine est un radical acétyle ou *tert*-butyloxycarbonyl, et le groupe protecteur de la fonction β-amino alcool est un radical isopropylidène ou benzylidène. X représente un atome d'halogène, de préférence brome ou iode, ou bien un radical perfluoroalkylsulfonate.

La réaction a s'effectue généralement en présence d'un dérivé du bore tel que le 9-borabicyclononane ou le disiamylborane au sein d'un solvant aromatique tel que le toluène ou bien d'un solvant éthéré tel que le THF, à une température comprise entre 10°C et la température d'ébullition du milieu réactionnel. Puis au milieu réactionnel est ajoutée une solution aqueuse d'un hydroxyde de métal alcalin tel que l'hydroxyde de sodium ou de potassium, suivi d'un complexe d'un métal de transition tel que le tétrakistriphénylphosphine de palladium ou bien le diphénylphosphinoferrocènyl de dichlorure de palladium, suivi de HetX. Le mélange résultant est chauffé à la température d'ébullition du milieu réactionnel.

La réaction de déprotection b s'effectue avec un agent de déprotection pour les composés pour lesquels Ra est un groupe protecteur de la fonction amine et Rb est un groupe protecteur de la fonction β-amino alcool par toutes les méthodes connues de l'homme de l'art et notamment celles décrites par T.W. GREENE, Protective groups in Organic Synthesis, J. Wiley-Interscience Publication (1991). De préférence lorsque le groupe protecteur de la fonction amine est un radical *tert*-butyloxycarbonyle, et le groupe protecteur de la fonction β-amino alcool est un radical isopropylidène ou benzylidène, cette réaction s'effectue au moyen d'un acide tel que l'acide chlorhydrique, en milieu aqueux, à une température voisine de 25° C. On utilise en général l'acide chlorhydrique 6N. Lorsque le groupe protecteur de la fonction amine est un radical acétyle, et le groupe protecteur de la fonction β-amino alcool est un radical isopropylidène ou benzylidène, cette réaction s'effectue au moyen d'un acide tel que l'acide chlorhydrique, en milieu aqueux, à une température proche du point d'ébullition du milieu réactionnel. On utilise en général l'acide chlorhydrique 6N.

La réaction c s'effectue par action du *tert*-butyl isothiocyanate, au sein d'un solvant inerte tel qu'un alcool aliphatique (1-4C) (méthanol, éthanol de préférence), en présence d'une amine tertiaire telle que la triéthylamine, à une température comprise entre 20°C et la température d'ébullition du milieu réactionnel.

Les composés de formule (I) sont isolés et peuvent être purifiés par les méthodes connues habituelles, par exemple par cristallisation, chromatographie ou extraction.

Les énantiomères des composés de formule (I) peuvent être obtenus par dédoublement des racémiques par exemple par chromatographie sur colonne chirale selon PIRCKLE W.H. et coll., asymmetric synthesis, vol. 1, Academic Press (1983) ou par formation de sels ou par synthèse à partir des précurseurs chiraux. Les diastéréoisomères peuvent être préparés selon les méthodes classiques connues (cristallisation, chromatographie ou à partir des précurseurs chiraux).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels suivants : benzènesulfonate, bromhydrate, chlorhydrate, citrate, éthanesulfonate, fumarate, gluconate, iodate, iséthionate, maléate, méthanesulfonate, méthylène-bis-b-oxynaphtoate, nitrate, oxalate, pamoate, phosphate, salicylate, succinate, sulfate, tartrate, théophyllinacétate et p-toluènesulfonate.

Les composés de formule (I) sont des inhibiteurs de NO-synthase inductible ou NO-synthase de type 2 (NOS-2) et sont ainsi utiles pour la prévention et le traitement des désordres liés à une production excessive de NO telles que la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie, le diabète, l'athérosclérose, la myocardite, l'arthrite, l'arthrose, l'asthme, le syndrome du colon irritable, la maladie de Crohn, la péritonite, le reflux gastro-oesophagien, l'uveite, le syndrome de Guillain-Barré, la glomerulo-néphrite, le lupus erythémateux, le psoriasis, la croissance de certaines formes de tumeurs comme par exemple les épithéliomes, les adénocarcinomes ou les sarcomes, et les infections par des bactéries intracellulaires ou extracellulaires, gram-plus ou gram-moins.

Leurs activités en tant qu'inhibiteurs de NOS-2 et NOS-3 ont été déterminées par la mesure de la conversion de [³H]-L-arginine en [³H]-L-citrulline par, respectivement, une fraction enzymatique NOS-2 extraite de poumons de rats ou de souris préalablement traités par des lipopolysaccharides (10 mg/kg i.p. 6 heures avant le recueil de tissu) et par une préparation commerciale de NOS-3 recombinante de boeuf.

Les composés ont été incubés pendant 20 à 30 minutes à 37°C en présence de 5 µM (pour activité NOS-2) ou 10 µM (pour activité NOS-3) de [³H]-L-arginine, 1 mM de NADPH, 15 µM de tétrabiopterine, 1µM de FAD, 0.1 mM de DTT dans un tampon HEPES (50 mM, pH 6,7) contenant 10 µg/ml de calmoduline et 1.25 mM de CaCl₂ lorsque l'activité NOS-3 a été mesurée. L'incubation a été arrêtée par addition de tampon HEPES froid (100 mM, pH 5,5) contenant 10 mM d'EGTA et 500 mg d'une résine cationique échangeuse d'ion (AG50W-X8, contre-ion : Na⁺) pour séparer la [³H]-L-arginine de la [³H]-L-citrulline. Après 5 min de décantation, la radioactivité restant dans la phase liquide a été mesurée dans un compteur à scintillation en présence d'un liquide scintillant approprié. Le rendement de la récupération de la L-[³H]citrulline formée a pu être estimée en utilisant de la L-[ureido-¹⁴C]-citrulline comme standard externe.
L'activité NOS-2 ou NOS-3 a été exprimée en picomole(s) de [³H]-L-citrulline formée par minute et par milligramme de protéine contenu dans le milieu réactionel.

Dans ce test sur l'enzyme NOS-2, la CI₅₀ des composés de formule (I) est inférieure ou égale à 10 µM.

La sélectivité est mesurée par le rapport CI₅₀ NOS-3 / CI₅₀ NOS-2. Cette sélectivité est supérieure à 30.

Les composés de formule (I) présentent une toxicité faible. Leur DL₅₀ est supérieure à 40 mg/kg par voie sous cutanée chez la souris.

L'exemple suivant illustre l'invention de manière non limitative.

### Exemple1 :

### (4R)-4-(2-Thién-3-yl-éthyl)-4,5-dihydro-1,3-thiazol-2-ylamine, chlorhydrate

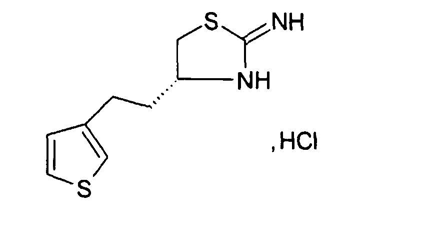

Un mélange 0,72 g de *N*-(*tert*-butyl)-*N*'-[(1R)-2-hydroxy-1-(2-thién-3-yl-éthyl)éthyl]-thiourée dans 20 cm³ d'une solution aqueuse d'acide chlorhydrique 5N est chauffé sous agitation magnétique à une température voisine de 100°C pendant 18 heures. Le milieu réactionnel est ensuite évaporé sous pression réduite (2 kPa) à une température voisine de 40°C et le résidu obtenu est repris dans 20 cm³ d'éthanol et de nouveau concentré selon les conditions décrites ci-dessus. Le résidu d'évaporation est trituré dans 5 cm³ d'éthanol, filtré, lavé par 2 fois 2 cm³ d'éthanol et par 2 fois 5 cm³ d'éther diéthylique. Le produit est séché dans l'étuve sous vide (10 Pa) à une température voisine de 20°C. On obtient 0,28 g de (4R)-4-(2-thién-3-yléthyl)-4,5-dihydro-1,3-thiazol-2-ylamine, chlorhydrate, sous forme d'un solide beige fondant aux environs de 150°C. Spectre de R.M.N. ¹H (300 MHz, (CD₃)₂SO d6, δ en ppm) : de 1,85 à 2,10 (mt : 2H) ; 2,70 (mt : 2H) ; de 3,30 à 3,45 (mt : 1H) ; 3,69 (dd, J = 11 et 7,5 Hz : 1H) ; 4,21 (mt : 1H) ; 7,05 (dd, J = 5 et 1,5 Hz : 1H) ; 7,26 (mt : 1H) ; 7,50 (dd, J = 5 et 3 Hz : 1H) ; 9,10 (mf: 1H) ; 9,61 (mf: 1H) ; 10,27 (s large : 1H).

### N-(tert-Butyl)-N'-[(1R)-2-hydroxy-1-(2-thién-3-yléthyl)éthyl]-thiourée

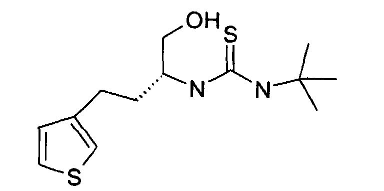

Une solution de 1,2 g de (2R)-2-amino-4-(3-thiényl)-1-butanol, chlorhydrate dans 40 cm³ d'éthanol, additionnée de 1,1 cm³ de *tert*-butylisothiocyanate et de 1 cm³ de triéthylamine est agitée magnétiquement sous atmosphère inerte à une température voisine de 20°C, puis chauffée pendant 20 heures à une température voisine de 50°C. Le milieu réactionnel est concentré sous pression réduite (2 kPa) à une température voisine de 50°C. Le résidu d'évaporation est purifié par chromatographie sous pression d'argon (70 kPa), sur une colonne de gel de silice (granulométrie 60-200 µm ; diamètre 3,6 cm ; hauteur 20 cm), en éluant par un mélange de cyclohexane-acétate d'éthyle (60/40 en volumes) et en recueillant des fractions de 30 cm³. Les fractions contenant le produit attendu sont réunies et évaporées sous pression réduite (2 kPa) à une température voisine de 40°C. On obtient 0,73 g de *N*-(*tert*-butyl)-*N*'-[(1R)-2-hydroxy-1-(2-thién-3-yléthyl)éthyl]thiourée, sous forme d'une huile incolore. Spectre de R.M.N ¹H (300 MHz, (CD₃)₂SO d6, δ en ppm) : 1,42 (s : 9H) ; de 1,60 à 1,95 (mt : 2H) ; 2,60 (t large, J = 8 Hz : 2H) ; 3,38 (mt : 1H) ; 3,50 (mt : 1H) ; 4,26 (mf: 1H) ; 4,80 (mf: 1H) ; 7,01 (dd, J = 5 et 1,5 Hz : 1H) ; de 7,15 à 7,25 (mt : 2H) ; 7,20 (s : 1H) ; 7,46 (dd, J = 5 et 3 Hz : 1H).

### (2R)-2-Amino-4-(3-thiényl)-1-butanol, chlorhydrate

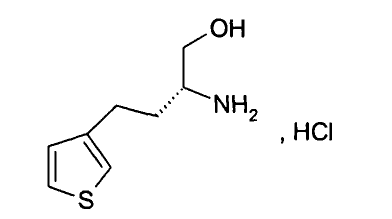

Une suspension de 1,8 g de 2,2-diméthyl-4-(2-thién-3-yl-éthyl)-oxazolidine-3-carboxylate de *tert*-butyle dans 5 cm³ d'une solution aqueuse d'acide chlorhydrique 5N et 5 cm³ de dioxanne est agitée à une température voisine de 20°C pendant 3 heures. Le mélange réactionnel est ensuite évaporé à sec sous pression réduite (2 kPa) à une température voisine de 40°C. On obtient 1,3 g de (2R)-2-amino-4-(3-thiényl)-1-butanol, chlorhydrate sous forme d'une huile épaisse. Spectre de R.M.N ¹H (300 MHz, (CD₃)₂SO d6, δ en ppm) : de 1,70 à 1,95 (mt : 2H) ; 2,70 (t large, J = 8 Hz : 2H) ; 3,05 (mt : 1H) ; 3,50 (dd, J = 11 et 6 Hz : 1H) ; 3,64 (dd, J = 11 et 4 Hz : 1H) ; 7,02 (dd, J = 5 et 1,5 Hz : 1H) ; 7,23 (d large, J = 3 Hz : 1H) ; 7,49 (dd, J = 5 et 3 Hz : 1H) ; 8,02 (mf : 3H).

### (4R)-2,2-Diméthyl-4-(2-thién-3-yl-éthyl)-oxazolidine-3-carboxylate de tert-butyle

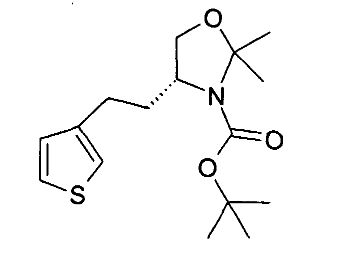

A une suspension de 1,5 g de (4R)-2,2-diméthyl-4-vinyl-oxazolidine-3-carboxylate de *tert*-butyle dans 33 cm³ de toluène, agitée sous atmosphère inerte, on ajoute 26,4 cm³ d'une solution de 9-borabicyclo-[3,3,1]-nonane. Le milieu réactionnel est chauffé à une température voisine de 70°C pendant 30 minutes. Le chauffage est retiré momentanément pour ajouter 5,3 cm³ d'une solution aqueuse de soude 5N dans 2 cm³ d'eau et au bout d'une minute, on ajoute 0,23 g de tétrakis(triphénylphosphine)palladium(0) et 0,81 cm³ de 3-bromo-thiophène. Le chauffage est ensuite poursuivi pendant 22 heures à une température voisine de 90°C. Après refroidissement du mélange réactionnel à une température voisine de 20°C, on ajoute 100 cm³ d'acétate d'éthyle. La phase organique est décantée, séchée sur du sulfate de magnésium, filtrée et concentrée sous pression réduite (2 kPa) à une température voisine de 40°C. Le résidu d'évaporation est purifié par chromatographie (70 kPa), sur une colonne de gel de silice (granulométrie 60-200 µm ; diamètre 3,6 cm ; hauteur 30 cm), en éluant par un mélange de cyclohexane-acétate d'éthyle (90/10 en volumes) et en recueillant des fractions de 60 cm³. Les fractions contenant le produit attendu sont réunies et évaporées à sec sous pression réduite (2 kPa) à une température voisine de 40°C. On obtient ainsi 1,8 g de (4R)-2,2-diméthyl-4-(2-thién-3-yl-éthyl)-oxazolidine-3-carboxylate de *tert*-butyle, sous forme d'une huile jaune.Spectre de R.M.N ¹H (400 MHz, (CD₃)₂SO d6, à une température de 373 K, δ en ppm) : 1,45 (s : 9H) ; 1,54 (s : 6H) ; de 1,75 à 2,10 (mt : 2H) ; 2,65 (mt : 2H) ; 3,75 (dd, J = 9 et 2 Hz : 1H) ; 3,85 (mt : 1H) ; 3,94 (dd, J = 9 et 6 Hz : 1H) ; 6,99 (d large, J = 5 Hz : 1H) ; 7,14 (mt : 1H) ; 7,40 (dd, J = 5 et 3 Hz : 1H).

Les compositions pharmaceutiques selon l'invention sont constitués par un composé de formule (I) ou un isomère ou un tautomère ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops ets élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention de la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie, le diabète, l'athérosclérose, la myocardite, l'arthrite, l'arthrose, l'asthme, le syndrome du colon irritable, la maladie de Crohn, la péritonite, le reflux gastro-oesophagien, l'uvéite, le syndrome de Guillain-Barré, la glomérulo-néphrite, le lupus érythémateux, le psoriasis, la croissance de certaines formes de tumeurs comme par exemple les épithéliomes, les adénocarcinomes ou les sarcomes, et les infections par des bactéries intracellulaires ou extracellulaires, Gram-plus ou Gram-moins.

Les doses dépendent de l'effet recherché, de la durée du traitement et de la voie d'administration utilisée; elles sont généralement comprises entre 1 mg et 100 mg par jour par voie orale pour un adulte avec des doses unitaires allant de 0,5 mg à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

## Revendications

1. Composés de formule (I) 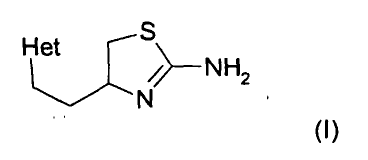 dans laquelle Het représente un radical 2-thiényle, 3-thiényle, 2-pyrimidyle, 5-pyrimidyle, 2-pyridyle, 3-pyridyle, 4-pyridyle, 2-thiazolyle, 4-thiazolyle ou 5-thiazolyle
leurs racémiques, énantiomères, diastéréoisomères et leurs mélanges, leurs tautomères et leurs sels pharmaceutiquement acceptables.

2. Composé selon la revendication 1 **caractérisé par le fait qu'**il est choisi parmi :
4-(2-pyridin-2-yléthyl)-4,5-dihydro-1,3-thiazol-2-ylamine
4-(2-pyridin-3-yléthyl)-4,5-dihydro-1,3-thiazol-2-ylamine
4-(2-pyridin-4-yléthyl)-4,5-dihydro-1,3-thiazol-2-ylamine
4-(2-thién-3-yléthyl)-4,5-dihydro-1,3-thiazol-2-ylamine
leur racémiques, énantiomères, diastéréoisomères et leurs mélanges, leurs tautomères et leurs sels pharmaceutiquement acceptables.

3. Composé selon l'une quelconque des revendications 1 à 2 **caractérisé par le fait qu'**il est choisi parmi :
(+)-(4R)-4-(2-pyridin-2-yléthyl)-4,5-dihydro-1,3-thiazol-2-ylamine
(+)-(4R)-4-(2-pyridin-3-yléthyl)-4,5-dihydro-1,3-thiazol-2-ylamine
(+)-(4R)-4-(2-pyridin-4-yléthyl)-4,5-dihydro-1,3-thiazol-2-ylamine
(4R)-4-(2-thién-3-yléthyl)-4,5-dihydro-1,3-thiazol-2-ylamine
leurs tautomères ainsi que leurs sels pharmaceutiquement acceptables.

4. Composé selon l'une quelconque des revendications 1 à 3 utilisé pour préparer un médicament.

5. Composition pharmaceutique **caractérisée par le fait qu'**elle comprend dans un milieu pharmaceutiquement acceptable, un composé selon l'une quelconque des revendicaitons 1 à 3.

6. Médicament selon la revendication 4 **caractérisé par le fait qu'**il contient au moins un composé selon l'une quelconque des revendications 1 à 3 pour son applicaiton thérapeutique dans le traitement des maladies dans lesquelles une production anormale de monoxyde d'azote (NO) par induction de NO-synthase inductible (NOS-2) est impliquée.

7. Médicament **caractérisé par le fait qu'**il contient au moins un composé selon l'une quelconque des revendications 1 à 3 pour son application thérapeutique dans le traitement de la maladie de Parkinson.

8. Procédé de préparation des composés de formule (I) tels que définis dans la revendication 1 **caractérisé en ce que** l'on cyclise un dérivé de formule : 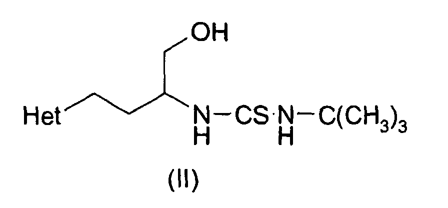 dans laquelle Het ont les mêmes significations que dans la revendication 1.

9. Procédé de préparation selon la revendication 8 **caractérisé en ce que** la cyclisation se fait en milieu acide à une température voisine de 100°C.

10. Procédé de préparation selon la revendication 9 **caractérisé en ce que** le milieu acide est préférentiellement de l'acide chlorhydrique 6N

11. Procédé de préparation des composés de formule (II) telle que définie à la revendication 8 et dans laquelle Het a la même signification qu'à la revendication 1 **caractérisé en ce qu'**on soumet un composé de formule (IIa) 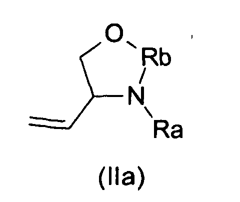 Ra un groupe protecteur de la formation amine et Rb est un groupement protecteur de la fonction β-amino alcool à l'action d'un dérivé de bore et X-Het afin d'obtenir un composé de formule (IIb) 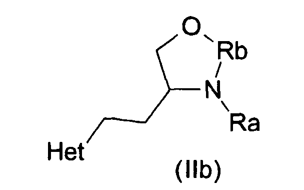 que l'on soumet à l'action d'un agent de déprotection afin d'obtenir un composé de formule (IIc) 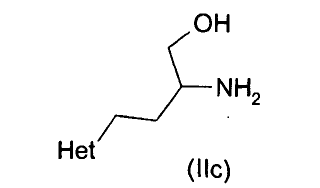 que l'on soumet à l'action du *tert*-butylisothiocyanate afin d'obtenir un composé de formule (II)

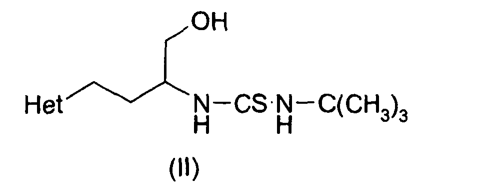

12. A titre de produits intermédiaires les composés *N*-(*tert*-Butyl)-*N*'-[(1R)-2-hydroxy-1-(2-thién-3-yléthyl)éthyl]-thiourée ; (2R)-2-Amino-4-(3-thiényl)-1-butanol, chlorhydrate ; (4R)-2,2-Diméthyl-4-(2-thién-3-yl-éthyl)-oxazolidine-3-carboxylate de *tert*-butyle

## Patentansprüche

1. Verbindungen der Formel (I) 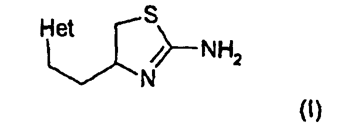 in der Het einen 2-Thienyl-, 3-Thienyl-, 2-Pyrimidyl-, 5-Pyrimidyl-, 2-Pyridyl-, 3-Pyridyl-, 4-Pyridyl-, 2-Thiazolyl-, 4-Thiazolyl- oder 5-Thiazolylrest darstellt,
ihre racemischen Gemische, Enantiomeren, Diastereomeren und ihre Gemische, ihre Tautomeren und ihre pharmazeutisch annehmbaren Salze.

2. Verbindung nach Anspruch 1, **dadurch gekennzeichnet, dass** sie ausgewählt ist aus:
4-(2-Pyridin-2-ylethyl)-4,5-dihydro-1,3-thiazol-2-ylamin
4-(2-Pyridin-3-ylethyl)-4,5-dihydro-1,3-thiazol-2-ylamin
4-(2-Pyridin-4-ylethyl)-4,5-dihydro-1,3-thiazol-2-ylamin
4-(2-Thien-3-ylethyl)-4,5-dihydro-1,3-thiazol-2-ylamin
ihren racemischen Gemischen, Enantiomeren, Diastereomeren und ihren Gemischen, ihren Tautomeren und ihren pharmazeutisch akzeptablen Salzen.

3. Verbindung nach einem der Ansprüche 1 bis 2, **dadurch gekennzeichnet, dass** sie ausgewählt ist aus:
(+)-(4R)-4-(2-Pyridin-2-ylethyl)-4,5-dihydro-1,3-thiazol-2-ylamin
(+)-(4R)-4-(2-Pyridin-3-ylethyl)-4,5-dihydro-1,3-thiazol-2-ylamin
(+)-(4R)-4-(2-Pyridin-4-ylethyl)-4,5-dihydro-1,3-thiazol-2-ylamin
(4R)-4-(2-Thien-3-ylethyl)-4,5-dihydro-1,3-thiazol-2-ylamin
ihren Tautomeren sowie ihren pharmazeutisch annehmbaren Salzen.

4. Verbindung nach einem der Ansprüche 1 bis 3, die zur Herstellung eines Medikaments verwendet wird.

5. Pharmazeutische Zusammensetzung, **dadurch gekennzeichnet, dass** sie in einem pharmazeutisch annehmbaren Medium eine Verbindung nach einem der Ansprüche 1 bis 3 umfasst.

6. Medikament nach Anspruch 4, **dadurch gekennzeichnet, dass** es wenigstens eine Verbindung nach einem der Ansprüche 1 bis 3 enthält, zur therapeutischen Anwendung bei der Behandlung der Krankheiten, in denen eine abnormale Stickstoffmonoxid- (NO-) Produktion durch Induktion der induzierbaren NO-Synthase (NOS-2) impliziert ist.

7. Medikament, das wenigstens eine Verbindung nach einem der Ansprüche 1 bis 3 enthält, zur therapeutischen Anwendung in der Behandlung der Parkinson-Krankheit.

8. Herstellungsverfahren der Verbindungen der Formel (I), wie sie in Anspruch 1 definiert sind, **dadurch gekennzeichnet, dass** man ein Derivat der Formel: 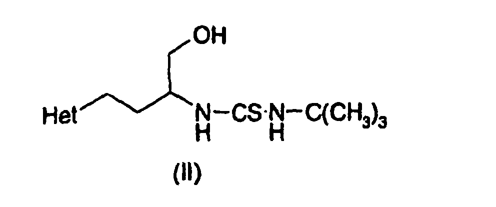 worin Het dieselben Bedeutungen wie in Anspruch 1 hat, cyclisiert.

9. Herstellungsverfahren nach Anspruch 8, **dadurch gekennzeichnet, dass** die Cyclisierung in saurem Medium bei einer Temperatur von ungefähr 100°C erfolgt.

10. Herstellungsverfahren nach Anspruch 9, **dadurch gekennzeichnet, dass** das saure Medium vorzugsweise 6 N Salzsäure ist.

11. Herstellungsverfahren für die Verbindungen der Formel (II), wie sie in Anspruch 8 definiert sind, und in der Het dieselbe Bedeutung wie in Anspruch 1 hat, **dadurch gekennzeichnet, dass** man eine Verbindung der Formel (IIa) 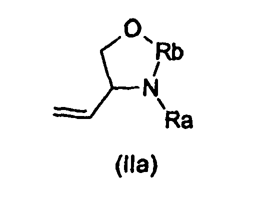 worin Ra eine Schutzgruppe der Aminformation ist und Rb eine Schutzgruppe der β-Aminoalkoholfunktion ist,
der Wirkung eines Borderivats und X-Het unterwirft, wobei eine Verbindung der Formel (IIb) erhalten wird 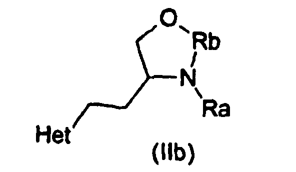 die man der Wirkung eines Entschützungsmittels unterwirft, um eine Verbindung der Formel (IIc) zu erhalten 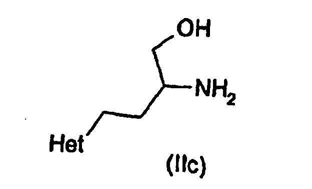 die man der Wirkung von tert-Butylisothiocyanat unterwirft, um eine Verbindung der Formel (II) zu erhalten:

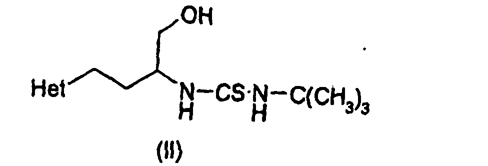

12. Verbindungen N-(tert-Butyl)-N'-[(1R)-2-hydroxy-1-(2-thien-3-ylethyl)ethyl]-thioharnstoff; (2R)-2-Amino-4-(3-thienyl)-1-butanol-Hydrochlorid; (4R)-2,2-Dimethyl-4-(2-thien-3-yl-ethyl)oxazolidin-3-carbonsäure-tert-butylester als Zwischenprodukte.

## Claims

1. Compounds of formula (I) 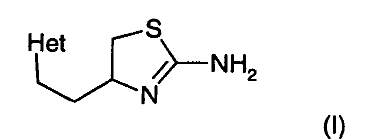 in which Het represents a 2-thienyl, 3-thienyl, 2-pyrimidyl, 5-pyrimidyl, 2-pyridyl, 3-pyridyl, 4-pyridyl, 2-thiazolyl, 4-thiazolyl or 5-thiazolyl radical
their racemic mixtures, enantiomers, diastereoisomers and mixtures thereof, their tautomers and their pharmaceutically acceptable salts.

2. Compound according to Claim 1 **characterized in that** it is selected from:
4-(2-pyridin-2-ylethyl)-4,5-dihydro-2,3-thiazol-2-ylamine
4-(2-pyridin-3-ylethyl)-4,5-dihydro-1,3-thiazol-2-ylamine
4-(2-pyridin-4-ylethyl)-4,5-dihydro-1,3-thiazol-2-ylamine
4-(2-thien-3-ylethyl)-4,5-dihydro-1,3-thiazol-2-ylamine
their racemic mixtures, enantiomers, diastereoisomers and mixtures thereof, their tautomers and their pharmaceutically acceptable salts.

3. Compound according to any one of Claims 1 to 2 **characterized in that** it is selected from:
(+)-(4R)-4-(2-pyridin-2-ylethyl)-4,5-dihydro-1,3-thiazol-2-ylamine
(+)-(4R)-4-(2-pyridin-3-ylethyl)-4,5-dihydro-1,3-thiazol-2-ylamine
(+)-(4R)-4-(2-pyridin-4-ylethyl)-4,5-dihydro-1,3-thiazol-2-ylamine
(4R)-4-(2-thien-3-ylethyl)-4,5-dihydro-1,3-thiazol-2-ylamine
their tautomers and their pharmaceutically acceptable salts.

4. Compound according to any one of Claims 1 to 3 used to prepare a medicament.

5. Pharmaceutical composition **characterized in that** it comprises in a pharmaceutically acceptable medium a compound according to any one of Claims 1 to 3.

6. Medicament according to Claim 4 **characterized in that** it contains at least one compound according to any one of Claims 1 to 3 for its therapeutic application in the treatment of diseases involving abnormal production of nitrogen monoxide (NO) by induction of inducible NO synthase (NOS-2).

7. Medicament **characterized in that** it contains at least one compound according to any one of Claims 1 to 3 for its therapeutic application in the treatment of Parkinson's disease.

8. Process for preparing compounds of formula (I) as defined in Claim 1 **characterized in that** a derivative of formula: 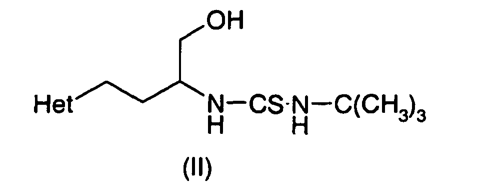 in which Het has the same meanings as in Claim 1 is cyclized.

9. Preparation process according to Claim 8 **characterized in that** the cyclization takes place in an acidic medium at a temperature in the region of 100°C.

10. Preparation process according to Claim 9 **characterized in that** the acidic medium is preferably 6N hydrochloric acid.

11. Process for preparing compounds of formula (II) as defined in Claim 8 and in which Het has the same meaning as in Claim 1 **characterized in that** a compound of formula (IIa) 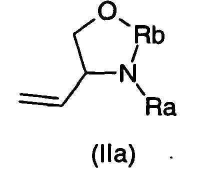 in which Ra is a protective group for the amine formation and Rb is a protective group for the β-amino alcohol function is subjected to the action of a boron derivative and X-Het so as to give a compound of formula (IIb) 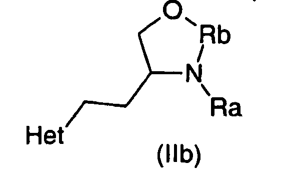 which is subjected to the action of a deprotection agent so as to give a compound of formula (IIc) 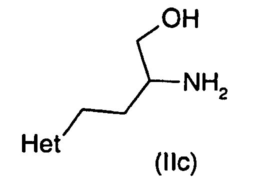 which is subjected to the action of *tert*-butyl isothiocyanate so as to give a compound of formula (II)

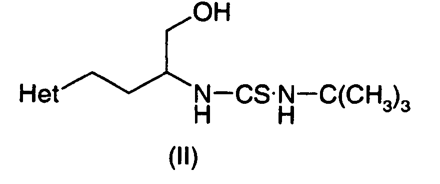

12. As intermediates, the compounds *N-(tert*-butyl)-*N*'-[(1R)-2-hydroxyl-1-(2-thien-3-ylethyl)ethyl]thiourea;
(2R)-2-amino-4-(3-thienyl)-1-butanol hydrochloride; *tert*-butyl (4R)-2,2-dimethyl-4-(2-thien-3-ylethyl)oxazolidine-3-carboxylate.
